Europäisches Patentamt

(19) European Patent Office

Office européen des brevets

(11) Publication number: **0 040 943**
**B1**

(12) **EUROPEAN PATENT SPECIFICATION**

(45) Date of publication of patent specification: **29.08.84**

(51) Int. Cl.³: **G 01 N 33/54,** G 01 N 33/56

(21) Application number: **81302195.3**

(22) Date of filing: **18.05.81**

(54) Improvements in or relating to support means.

(30) Priority: **27.05.80 GB 8017335**

(43) Date of publication of application:
**02.12.81 Bulletin 81/48**

(45) Publication of the grant of the patent:
**29.08.84 Bulletin 84/35**

(84) Designated Contracting States:
**DE FR IT**

(56) References cited:
**DE-A-2 170 506**
**DE-A-2 824 164**
**FR-A-2 277 563**
**FR-A-2 377 629**
**FR-A-2 394 088**
**US-A-2 956 686**
**US-A-4 111 754**

(73) Proprietor: **Secretary of State for Social Services in Her Britannic Majesty's Government of the United Kingdom of Great Britain and Northern Ireland Alexander Fleming House Elephant & Castle London SE1 6BY (GB)**

(72) Inventor: **Bunce, Roger Abraham**
**117 Berberry Close**
**Bourneville Birmingham (GB)**
Inventor: **Studer, Rolf Otto**
**124 Grenzascherstrasse**
**Basle (CH)**

(74) Representative: **Gunning, William John et al**
**Procurement Executive, Ministry of Defence Patents 1A(4), Room 1932 Empress State Building Lillie Road London SW6 1TR (GB)**

Courier Press, Leamington Spa, England.

## Description

This invention relates to supports for small objects, more especially plastic forms, e.g. spheres, which are used as a base for diagnostic coatings to react, in a process of analysis, with biological fluids or solution thereof.

Certain chemicals, for example the globulin fraction of antiserum against alpha-fetoprotein may be absorbed or covalently bonded to solid phases such as the plastic, polystyrene. This is made use of in the determination of, for example, the concentration of alpha-fetoprotein in human blood serum. It has been usual to bring the chemical and the solid phase onto which it is to be absorbed or covalently bonded into contact with each other manually. Typically, two different types of manual procedures have been used; either a plastics solid form has been dipped or immersed in the appropriate coating solution, or the inside surface of a plastics tube-like container has been coated by filling with the coating solution and then emptying. The coated forms may then be used to determine the concentration of an analyte in a sample. Each stage of this analysis has required a manual transfer which is time consuming, leads to poor precision, causes problems in maintaining patient identity, and is expensive.

Plastic forms have been supported, in solution for coating, washing, or reaction in analysis, by stems which in turn have been supported from above. (See, for example, French Patent Specification No. 2377629, in particular Fig. 12.) From the point of view of expense and convenience in manufacture and packing, and, at least, of convenience in use, it is preferable to do away with such supporting stems and use, for example, a plain sphere as the plastics form.

It has been conventional in various industries, for coating, washing or draining of objects, to support them singly, or in numbers in a perforated container. However, the problems of chemical analysis to which, in particular, plastics forms are applied, requires minimal contact of support with plastics form. This allows maximal drainage on withdrawal from the fluid. It is also highly desirable that the plastics form be able to move in relation to the support. In medical work, in particular, maintenance of patient identity requires that each plastics form has its own individual support. The present invention meets these requirements to a very large extent and provides a means for supporting plastics forms so that they may easily be inserted into apparatus, used for analysis, and then removed from the apparatus.

According to the invention, support means for supporting more than six plastics forms of the kind used as a base for diagnostic coatings, said forms being e.g. spherical, comprises a substantially planar member having more than six siting means in the forms of holes therein; and is characterised by having fixed thereto, one to each siting means, cage-like structures extending from said planar member substantially normal thereto, each cage-like structure having at its end remote from said planar member a constriction or equivalent means whereby, in use, a plastics forms inserted in any one of said cage-like structures, and allowed to fall therein, is retained at the end thereof remote from said planar member.

Conveniently each cage-like structure is tapered in diameter away from the planar member and may have the end thereof remote from said planar member conforming substantially in shape to that of a plastics form required to be supported in said cage-like structure.

Each cage-like structure is preferably a filament formed in at least one helix fixed at one end in a respective siting means and constricted at the end thereof remote from said planar member. The filament may be a metal wire, for example stainless steel, and may further be covered with a layer of inert material, for example polytetrafluoroethylene. The helix may be reinforced by an external shrunk-on or moulded sleeve.

The planar member may conveniently be provided with locating holes of characteristically unsymmetrical cross section to mate, in use, with pegs of a complementary shape on a coacting abutment. Alternatively the planar member may have locating pegs of characteristically unsymmetrical cross section or arrangement to mate with holes of complementary shape in an abutment.

In a modified construction, each cage-like structure is a basket comprising a plurality of filaments extending from said planar member substantially normal thereto, and shaped at the end of the structure remote from said planar member so that, in use, an object inserted in such basket, and allowed to fall therein, is retained at the thereof remote from said planar member. Each filament may be a metal wire, such as stainless steel, and can be coated with an inert material, for example polytetrafluoroethylene of a polyurethane varnish.

The invention will be further described, by way of example only, with reference to the accompanying drawings in which

Fig. 1 is a trimetric general view of a support means according to the invention.

Fig. 2 is a scrap view of part of the support means on a larger scale

Fig. 3 is a scrap view of part of the support means showing locating means for locating the support means on an abutment.

Fig. 4 illustrates one optional means for rendering a part of the support means less flexible by reinforcing it.

Fig. 5 illustrates a cage-like structure in the form of a basket

Fig. 6 illustrates a modified basket construction

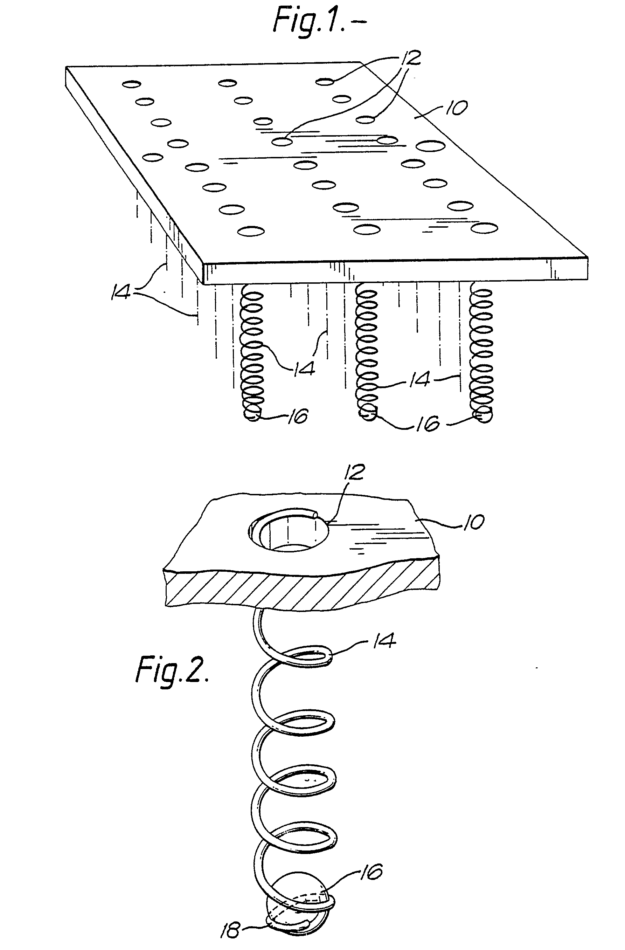

The support means comprises (Fig. 1) a planar member in the form of a flat plate 10 in which are a number of holes 12, of which, in this embodiment there are twenty four, although up to a hundred or more such holes may be provided. In this embodiment each hole 12 has a female thread, and each is provided with a helix 14, of which three only are shown in Fig. 1, to avoid complication of the drawing. The remaining helices are indicated diagrammatically by their centre lines. Each complete helix is shown supporting a plastic solid form 16 of spherical shape. A form 16 is, in use, inserted through a hole 12 in the plate 10 and (the plate being horizontal) falls to the end of the helix 14; that is to say the lower or remote end. Fig. 2 indicates how the plastics solid form 16 is prevented from dropping through the lower end of the helix. Substantially the last half turn at the lower end of the helix is constricted by being shaped, as indicated at 18, and turned inwards, slightly towards the axis of the helix. Alternatively the last half turn may be bent in to a somewhat smaller radius or curvature then the remainder of the helix. In either case the plastics form 16 is permitted to rest loosely at the lower end of the helix, having minimal contact therewith.

In this embodiment of the invention, the filament from which the helices are made is basically stainless steel wire. In order to avoid reaction with fluids used in analysis, or in coating or washing the forms 16, the stainless steel wire is provided with an inert protective surface layer. For this purpose polyurethane varnish, and polytetrafluoroethylene have been found to be adequately effective. The protective layer may be provided as a thin-walled tube slid onto the wire and sealed at the lower end, or the wire may be sprayed with, or dipped in, the inert material which is then allowed to harden or set. Alternatively a filament consisting wholly of suitably inert plastics material may be used.

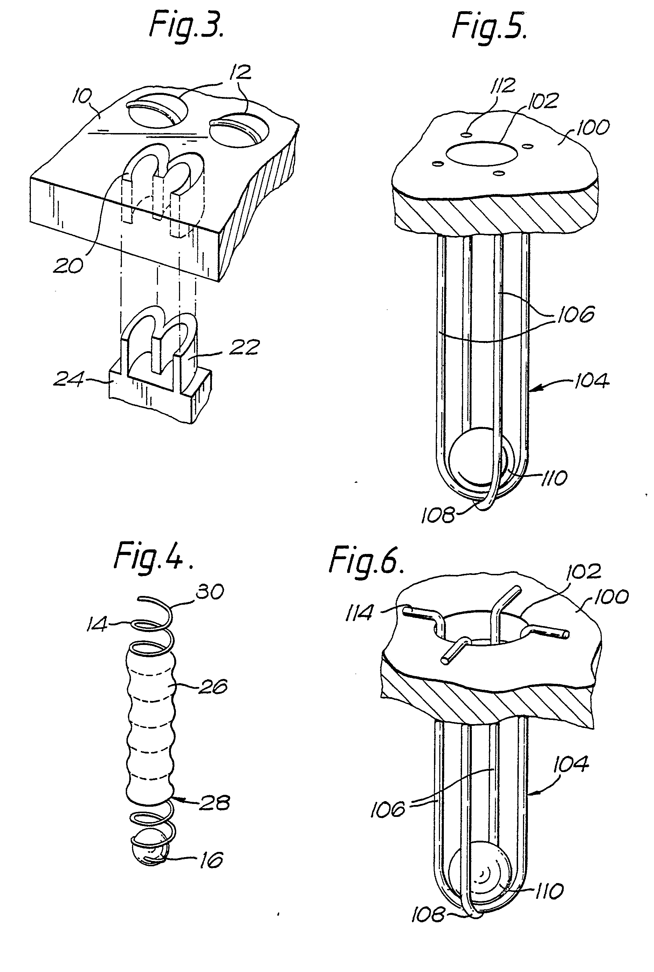

Especially in analysis for medical diagnostic purposes it may be very important to ensure patient identity between a particular plastics form 16, or set of such forms, and a or a set of containers of fluid of given composition. Maintenance of identity can be assisted by making the plate 10 with locating holes (20 in Fig. 3) of characteristically unsymmetrical cross section, to mate with pegs 22 of complementary shape on a coacting abutment. The abutment 24 may be part of an analysis apparatus. In an alternative construction, not separately illustrated, the peg 22 may be part of the plate 10 and the hole 20 be part of the abutment 24.

So that access of fluid to a plastics form 16 may be as free as possible, the filament of which the helices 14 are constructed should be of as small cross section as practicable. This may give a helix which is too flexible, and which responds too readily to, say, currents of washing fluid; even to the extent of releasing the form 16 from the helix. It has been found that a helix made from a small cross section filament can be rendered less flexible by enclosing part of the length of the helix in a shrunk-on sleeve of heat shrinkable membrane, usually a plastics material, as illustrated in Figure 4, at 26. The sleeve is confined to that part of the helix (about 2/3 to 3/4 of the length) near to the plate 10 and away from the part occupied by the plastics form 16, so that circulation of fluid around said form is not substantially hindered. The sleeve can be shrunk onto the helix by application of a hot air gun; but this can give rise to excessive shrinkage, constricting the helix and making it difficult or impossible to insert a plastic form. It has been found that more consistent results can be obtained by immersing the helix and sleeve in very hot water. Alternatively the sleeve may be moulded. It may be attached to the plate 10.

As already described, the helix 14 may be fixed in the plate 10 by what, in use, is its upper end, as shown in Figs. 1 and 2. Alternatively the helix may be fixed in the plate 10 at an intermediate location along its length, e.g. at the location 28 indicated in Fig. 4. If desired, the sleeve 26 may be used in fixing the helix in the plate 10, and the sleeve may also be extended to the end 30 of the helix, for easier insertion of an object 16 in said helix. The sleeve need not then necessarily be shrunk on to the helix, but merely enclose it, since, being, in use, above plate 10 it cannot slip off the helix.

A modified form of cage-like structure may be employed. Referring to Fig. 5 the planar member in the form of a plate is indicated by 100 and one of a number of holes therethrough — up to a hundred or more — is indicated by 102. The cage or basket, indicated generally by 104, is elongated, and extends from the plate normal thereto, and, in use, in a downward direction therefrom, as illustrated. The basket comprises four filaments 106, substantially parallel with one another over the greater part of their length, and mutually joined at 108, thereby supporting a spherical plastics form 110. The filaments 106 are fixed in the plate 100 by being made a close fit in bores, indicated by 112, in plate 100.

The filaments making up the cage 104 are made of a material which will not react with any liquid in which it is required to support the plastics forms 110. This may be done by making the filaments of a solid inert material or by covering say a metal wire with an inert coating material.

Fig. 6 illustrates an alternative way of fixing the basket 104 in the plate 100. Here the ends of the filaments 106 adjacent the plate 100 have an outwardly bent shape with the portions 114 substantially at right angles to the rest of the basket, so that they engage the upper surface, in use, of the plate 100, preventing the basket from passing through the plate. This construction has the advantage that, if required, a basket can readily be removed from the plate 100.

Use of the support means is facilitated by constructing the cage-like structures, whether of helix- or basket-form, so as to be tapered in diameter away from the planar member, at least in the portion directly adjacent said member. This facilitates the insertion of an array of cage-like structures into a corresponding array of containers, such as test tubes, without danger of one or more structures becoming fouled by an adjacent container.

Effective drainage of liquid from the support means, after immersion of a supported object, is desirable, in that it reduces contamination by one liquid or another in which the object may subsequently be immersed. It will usually be known what size and shape of object is to be held by the support means; a very common object being a spherical bead. Liquid tends to be retained between the object and the remote end of the cage-like structure, whether of helix- or basket-form. This retention is reduced by making said remote end conform substantially in shape, e.g. in radius of curvature, to the shape of the object, so that minimal gap exists between object and structure.

Controlling the speed at which the supported objects are withdrawn from immersion can also assist in reducing retention of liquid by the cage-like structures. The optimal withdrawal speed is in the order of 5 to 10 mm/sec.

Surface tension at the free surface of the liquid can be assisted to remove excess liquid by making the filament or filaments of the cage-like structures intersect the liquid surface at as steep an angle as possible. In the case of the helix-form, this involves using a steep helix angle, as steep as possible consistent with not permitting a supported object to escape laterally between turns of the helix. Such lateral escape can be avoided, while achieving a steep helix angle, by making the cage-like structure of two or more coaxial helices of the same diameter.

## Claims

1. Support means for supporting more than six plastics forms (16, 110) of the kind used as a base for diagnostic coatings, said forms being e.g. spherical; including a substantially planar member (10) having more than six siting means in the form of holes therein; characterised by having fixed thereto, one to each siting means, cage-like structures (14, 104) extending from said planar member substantially normal thereto, each cage-like structure (14, 104) having at its end (18, 108) remote from said planar member a constriction or equivalent means whereby, in use, a plastics form (16, 110) inserted in any one of said cage-like structures, and allowed to fall therein, is retained at the end thereof remote from said planar member (10).

2. Support means according to claim 1 characterised in that each cage-like structure (14) is tapered in diameter away from the planar member (10).

3. Support means according to claim 1 or claim 2 characterised in that the end (18) of each cage-like structure (14) remote from the planar member (10) conforms substantially in shape to that of a plastics form (16) required to be supported in said cage-like structure.

4. Support means according to any one of the preceding claims characterised in that each cage-like structure is at least one helix (14), formed from a filament, fixed at one end in a respective siting means (12) and constricted at the end thereof remote from said planar member.

5. Support means according to any one of claims 1 to 3 characterised in that each cage-like structure is a basket (104) comprising a plurality of filaments (106) extending from said planar member substantially normal thereto, and shaped at the end (108) of the structure remote from said planar member so that, in use, a plastics form (110) inserted in such basket (104), and allowed to fall therein, ia retained at the end thereof remote from said planar member.

6. Support means according to claim 4 or claim 5 characterised in that each filament (106) is a metal wire.

7. Support means according to claim 6 characterised in that the metal is stainless steel.

8. Support means according to claim 6 or claim 7 characterised in that the metal wire is covered with a layer of inert material.

9. Support means according to any one of claims 4 and 6 to 8 characterised in that the helix (14) is reinforced by an external sleeve (26).

10. Support means according to any one of the preceding claims characterised in that the planar member (10) has locating holes (20) of characteristically unsymmetrical cross section to mate, in use, with pegs (22) of complementary shape on a coacting abutment (24).

11. Support means according to any one of claims 1 to 9 characterised in that planar member (10) has locating pegs of characteristically unsymmetrical cross section of arrangement to mate with holes of a complementary shape in a coacting abutment.

## Revendications

1. Moyen de support destiné à supporter plus de six objets en forme en matière plastique (16, 110) du type utilisé comme base pour des revêtements diagnostiques, lesdits objets en forme étant par exemple sphériques, comprenant un organe sensiblement plan (10) comportant plus de six moyens de réception se présentant sous la forme de trous dans cet organe, caractérisé en ce qu'il comprend, fixé à lui à raison de un pour chaque moyen de réception, des structures en forme de cages (14, 104) s'étendant à partir dudit organe plant et sensiblement perpendiculairement à celui-ci, chaque structure en forme de cage (14, 104) ayant à son extrémité (18, 108) qui est éloignée dudit organe plan un

resserrement ou un moyen équivalent par lequel, en utilisation, un objet en forme en matière plastique (16, 110) inséré dans l'une quelconque desdites structures en forme de cages, et que l'on laisse tomber à l'intérieur, est retenu à son extrémité éloignée dudit organe plan (10).

2. Moyen de support selon la revendication 1, caractérisé en ce que en ce que chaque structure en forme de cage (14) est de forme évasée dont le diamètre diminue en s'éloignant de l'organe plan (10).

3. Moyen de support selon la revendication 1 ou 2, caractérisé en ce que chaque structure en forme de cage (14) présente à son extrémité qui est éloignée dudit organe plan (10) une forme qui est sensiblement celle d'un objet en forme en matière plastique (16) qui doit être supporté dans ladite structure en forme de cage.

4. Moyen de support selon l'une quelconque des revendications précédentes, caractérisé en ce que chaque structure en forme de cage est constituée par au moins un élément hélicoïdal, constitué par un filament fixé à une extrémité dans un moyen de réception respectif (12) et resserré à l'extrémité qui est éloignée dudit organe plan.

5. Moyen de support selon l'une quelconque des revendications 1 à 3, caractérisé en ce que chaque structure en forme de cage est un panier (104) comprenant plusieurs filaments (106) s'étendant à partir dudit organe plan et sensiblement perpendiculairement par rapport à celui-ci, et conformé à l'extrémité (108) de la structure qui est éloignée dudit organe plan de manière qu'en utilisation, l'objet en forme en matière plastique (110) inséré dans un tel panier (104) et que l'on laisse tomber à l'intérieur soit retenu à son extrémité éloignée dudit organe plan.

6. Moyen de support selon l'une quelconque des revendications 4 ou 5, caractérisé en ce que chaque filament (106) est un fil métallique.

7. Moyen de support selon la revendication 6, caractérisé en ce que le métal est l'acier inoxydable.

8. Moyen de support selon l'une quelconque des revendications 6 ou 7, caractérisé en ce que le fil métallique est recouvert d'une couche d'un matériau inerte.

9. Moyen de support selon l'une quelconque des revendications 4 et 6 à 8, caractérisé en ce que l'élément hélicoïdal (14) est renforcé par un manchon externe (26).

10. Moyen de support selon l'une quelconque des revendications précédentes, caractérisé en ce que l'organe plan (10) comprend des trous de positionnement (20) dont la section transversale a une asymétrie caractéristique qui se conjugue, en utilisation, avec des chevilles (22) de forme complémentaire montées sur une butée associée (24).

11. Moyen de support selon l'une quelconque des revendications 1 à 9, caractérisé en ce que l'organe plan (10) comprend des chevilles de positionnement de section transversale ou de disposition ayant une asymétrie caractéristique pour se conjuger avec des trous de forme complémentaire pratiqués dans une butée associée.

**Patentansprüche**

1. Trägermittel,
— das mehr als sechs, z.B. kugelförmige, Kunststoffformen (16, 110) der Art, die als Träger für Diagnosebeschichtungen benutzt werden, hält,
— das ein im wesentlichen ebenes Element (10) aufweist, das mehr als sechs Positionierungsmittel in Form von Löchern aufweist, dadurch gekennzeichnet, daß daran zu je einem pro Positionierungsmittel befestigt sind:
— käfigartige Anordnungen (14, 104), die sich von dem ebenen Element im wesentlichen senkrecht aus erstrecken,
— jede käfigartige Anordnung (14, 104) an ihrem, von dem ebenen Element abgelegenen Ende (18, 108) eine Einengung oder gleichwirkende Mittel aufweist, wodurch eine, bei der Benutzung in eine der käfigartigen Anordnungen eingeführte und darin fallengelassene Kunststofform (16, 110) an dessen von dem ebenen Element (10) abgelegenen Ende zurückerhalten wird.

2. Trägermittel nach Anspruch 1, dadurch gekennzeichnet, daß sich jede käfigartige Anordnung (14) im Durchmesser mit zunehmenden Abstand von dem ebenen Element (10) verjüngt.

3. Trägermittel nach einem der Ansprüche 1 oder 2, dadurch gekennzeichnet, daß das von dem ebenen Element (10) abgelegene Ende (18) jeder käfigartigen Anordnung (14) in etwa in der Form mit der in der käfigartigen Anordnung zu halternden Kunststofform (16) übereinstimmt.

4. Trägermittel nach einem der vorstehenden Ansprüche, dadurch gekennzeichnet, daß jede käfigartige Anordnung wenigstens aus einer Spirale (14) besteht, die aus einem Draht geformt ist, mit einem Ende in einem zugehörigen Positionierungsmittel (12) befestigt ist und sich an dem von dem ebenen Element abgelegenen Ende verjüngt.

5. Trägermittel nach einem der Ansprüche 1 bis 3, dadurch gekennzeichnet, daß die käfigartige Anordnung ein Korb (104) ist, der mehrere Drähte (106) aufweist, die sich von dem ebenen Element im wesentlichen senkrecht aus erstrecken, und die an dem von dem ebenen Element abgelegenen Ende (108) so geformt sind, daß, während des Gebrauchs eine in solch einen Korb (104) eingefügte und fallengelassene Kunststofform (110) an dem von dem ebenen Element abgelegenen Ende des Korbs zurückgehalten wird.

6. Trägermittel nach einem der Ansprüche 4 oder 5, dadurch gekennzeichnet, daß jeder Draht (106) ein Metalldraht ist.

7. Trägermittel nach Anspruch 6, dadurch gekennzeichnet, daß das Metall rostfreier Stahl ist.

8. Trägermittel nach einem der Ansprüche 6 oder 7, dadurch gekennzeichnet, daß der Metalldraht mit einer Schicht aus inertem Material beschichtet ist.

9. Trägermittel nach einem der Ansprüche 4 und 6 bis 8, dadurch gekennzeichnet, daß die Spirale (14) mit einer äußeren Hülse (26) verstärkt ist.

10. Trägermittel nach einem der vorhergehenden Ansprüche, dadurch gekennzeichnet, daß das ebene Element (10) Positionierungslöcher (20) mit typischerweise unsymmetrischer Querschnittsfläche aufweist, die während des Gebrauchs mit eine komplementäre Form aufweisenden Zapfen (22) auf einem mitwirkenden Widerlager (24) zusammenpassen.

11. Trägermittel nach einem der Ansprüche 1 bis 9, dadurch gekennzeichnet, daß das ebene Element (10) Positionierungszapfen mit typischerweise unsymmetrischer Querschnittsfläche oder Anordnung aufweist, die mit einer komplementäre Form aufweisenden Löchern in einem mitwirkenden Widerlager zusammenpassen.

0 040 943

Fig.1.—

Fig.2.

# Fig.3.

10

12

20

22

24

# Fig.4.

30

14

26

28

16

# Fig.5.

112

102

100

106

104

110

108

# Fig.6.

102

100

114

104

106

110

108